# EUROPEAN PATENT APPLICATION

(11) **EP 4 840 442 A1**
(43) Date of publication of application: **18.11.2026**
(21) Application number: 26177294.1
(22) Date of filing: 07.05.2026
(51) Int. Cl.: A61K 9/48, A61K 31/4045, A61K 31/4415, A61K 36/53, A61K 36/736, A61P 25/22

(54) **COMPOSITION IN THE FORM OF A CAPSULE-IN-CAPSULE COMPRISING MELATONIN, LAVENDER ESSENTIAL OIL AND SOUR CHERRY EXTRACT**

(30) Priority: 16.05.2025 IT 202500011042
(71) Applicant: Biodue S.p.A., 50028 Barberino Tavarnelle (FI) (IT)
(72) Inventor: Pellizzato, Marzia, 31050 Vedelago TV (IT); Colletti, Alessandro, 14042 Calamandrana AT (IT)
(74) Representative: Perani & Partners S.p.A.

(57) **Abstract**

A composition comprising melatonin, essential oil of lavender and black cherry extract comprising procyanidin B2; wherein: the composition is in capsule-in-capsule form, wherein an inner gastro-resistant capsule is arranged inside a non-gastro-resistant outer capsule; the non-gastro-resistant outer capsule comprises a mixture comprising the essential oil of lavender, melatonin and suitable excipients and/or diluents; the inner gastro-resistant capsule comprises a mixture comprising the black cherry extract, a gelling agent selected from the group consisting of: gellan gum, carrageenan, pectin, and mixtures of the foregoing, preferably the gelling agent comprises or consists of carrageenan, and suitable excipients and/or diluents.

The composition according to the invention for use in the treatment or prevention of mild insomnia, sleep disorders and anxiety disorders.

## Description

### FIELD OF THE INVENTION

The present invention is in the field of nutraceutical formulations or food supplements for the treatment of sleep disorders, anxiety disorders, for improving sleep quality and for relaxation.

### STATE OF THE ART

Sleep problems are extremely widespread in the population and can derive from multiple factors, including stress, anxiety, light pollution, the use of electronic devices before sleeping, and alterations of the circadian rhythm due to work shifts or irregular lifestyles.

One of the main problems that compromise sleep quality are nocturnal awakenings, which reduce the continuity of rest and can lead to a feeling of tiredness and reduced lucidity during the day.

Among the most used active ingredients in food supplementation to promote sleep are melatonin and essential oil of lavender, thanks to their relaxing and circadian rhythm-regulating properties.

Melatonin is a hormone capable of acting as a regulator of the sleep-wake cycle. The intake of melatonin contributes to the induction of sleep and formulations that allow for its rapid release ensure a more immediate effect.

The essential oil of lavender possesses relaxing, anxiety-reducing, and sleep-inducing properties and can act in combination and simultaneously with melatonin.

Furthermore, in the context of clinical research on the antioxidant properties of the cherry, an improvement in sleep quality reported by subjects included in study [1] was found. This result has stimulated further investigation, directing research towards the identification of a possible relaxing potential of the cherry.

This improved sleep quality could be attributed to the presence of a procyanidin, B2, a particularly abundant flavonoid in cherries. Further studies [2] have confirmed that procyanidin B2 could act as an agent capable of promoting relaxation [3], with positive effects on sleep quality, promoting a deeper and uninterrupted rest, also thanks to the presence of a plant-derived melatonin intrinsic to the phytocomplex of the cherry.

### Problem of the known art

However, both melatonin and essential oil of lavender have some limitations.

Melatonin has a variable bioavailability and rapid pharmacokinetics, with a half-life in the blood of about 40-60 minutes, which means that its effect can wear off quickly, making it less effective in maintaining sleep throughout the night.

The essential oil of lavender, despite having anxiolytic and sedative properties thanks to volatile compounds contained therein such as linalool and linalyl acetate, has a limited assimilation and a fast metabolism, which can reduce the duration of its relaxing effect.

Considering this, it is evident how the solutions of the state of the art, consisting of nutraceutical formulations comprising melatonin and essential oil of lavender, can only favor and accelerate the initial phase of sleep induction, without however preventing nocturnal awakenings, prolonging the deep sleep phase, guaranteeing uninterrupted rest, and comprehensively improving sleep quality.

Procyanidin B2, also present in cherries, presents problems related to a limited bioavailability due to its poor solubility and difficulty of absorption at the intestinal level.

Therefore, there is a need for a technical solution capable of exploiting the potential of melatonin and essential oil of lavender in inducing sleep, and at the same time combining it with the relaxing and sleep quality-improving properties of procyanidin B2 present in cherries, overcoming the problems of each active ingredient related to bioavailability and pharmacokinetics.

### SUMMARY OF THE INVENTION

The object of the invention is a composition comprising melatonin, essential oil of lavender and black cherry extract comprising procyanidin B2; wherein: the composition is in capsule-in-capsule form, wherein an inner gastro-resistant capsule is arranged inside a non-gastro-resistant outer capsule; the non-gastro-resistant outer capsule comprises a mixture comprising the essential oil of lavender, melatonin and suitable excipients and/or diluents; the inner gastro-resistant capsule comprises a mixture comprising the black cherry extract, a gelling agent selected from the group consisting of: gellan gum, carrageenan, pectin, and mixtures of the foregoing, preferably the gelling agent comprises or consists of carrageenan, and suitable excipients and/or diluents.

Further objects of the present invention are: the composition according to the invention for use in the treatment or prevention of mild insomnia, sleep disorders and anxiety disorders; the non-therapeutic use of the composition according to the invention for promoting relaxation, decreasing stress, improving sleep quality, and promoting psychophysical well-being.

### Advantages of the invention

The present invention intends to respond to the need for compositions or nutraceutical formulations for improving the overall quality of sleep. To this end, the Applicant has developed a composition in capsule-in-capsule form that allows for the immediate release of melatonin and essential oil of lavender to promote sleep, and the subsequent controlled release of the cherry extract in order to promote relaxation, minimize nocturnal awakenings, and improve the overall quality of sleep.

Advantageously, the composition of the invention allows to:
- rapidly release essential oil of lavender and melatonin, exploiting their simultaneous action and creating an immediate effect that promotes relaxation and sleep, without interfering with the gradual release of procyanidin B2;
- improve the bioavailability of natural active ingredients, with particular reference to the black cherry extract comprising procyanidin B2; moreover, in the case the inner capsule contained essential oil of lavender, melatonin or other active ingredients with comparable properties, the same procyanidin B2, thanks to its antioxidant properties, can improve the bioavailability of these further active ingredients, influencing their absorption by acting not only as an active ingredient but also as a vehicle for said bioactive molecules and improving their solubility and absorption in the intestine. Indeed, procyanidins are able to interact with cell membranes and to modulate the transport of molecules through the digestive system, enhancing the efficacy of other active ingredients [4, 5];

- improve the therapeutic efficacy of antioxidant active ingredients of a polyphenolic nature, such as for example procyanidin B2, thanks to the use of controlled-release gastro-resistant capsules;
- ensure that the black cherry extract is not released prematurely in the stomach, where it could be degraded, but only when it reaches the alkaline pH of the intestine, where absorption is optimal;
- ensure a controlled release of the black cherry extract and of other active ingredients eventually present in the gastro-resistant inner capsule by means of gelling agents of natural origin which, once the intestine is reached, allow the formation of a matrix that retains procyanidin B2 and favors its slow dissolution in the enteric tract, also improving its bioavailability;
- avoid the use of complex microencapsulation technologies, by exploiting the use of natural gelling agents;
- obtain a complete and optimized therapeutic effect through the combination of rapid release of relaxing ingredients (essential oil of lavender and melatonin) and gradual release of procyanidin B2;
- counteract oxidative stress thanks to the antioxidant action of procyanidin B2, whose gradual release allows to fully exploit its anti-inflammatory and antioxidant effects.

The Applicant also wishes to emphasize that the present invention allows to advantageously overcome the difficulties of absorption at the intestinal level of procyanidin B2 thanks to two main factors:
- firstly, the gastro-resistance of the inner capsule comprising the black cherry extract which, in turn, comprises procyanidin B2, allows to avoid the hepatic degradation of the latter due to the first-pass hepatic effect;
- secondly, the anticipated dissolution of the non-gastro-resistant outer capsule leads to the release in the stomach of the oily phase comprising the active ingredients, causing the activation of lipases at the intestinal level. Procyanidin B2, arriving intact directly in the intestine, thus finds a favorable environment for emulsification thanks to the lipases present and, consequently, is much better absorbed compared to the simple case in which it is administered orally, even in the event of a gastro-protected administration (in the absence of a simultaneous pre-administration of a liquid phase).

### DETAILED DESCRIPTION OF THE INVENTION

For the purposes of the present invention, the definition "comprising" does not exclude the presence of further components/steps, in addition to those expressly listed after said definition.

For the purposes of the present invention, the definitions "consisting of" or "constituted by" instead exclude the presence of components/steps not expressly listed.

It should be noted that, for the purposes of the present invention, the essential oil of lavender can also be indicated with the expression "lavender oil" with the same meaning.

### Composition

By capsule-in-capsule, or *caps-in-caps,* is meant a pharmaceutical form that provides for the presence of a capsule of reduced dimensions inside another capsule of larger dimensions. The capsule-in-capsule can present in different dimensions, forms and colors and the content of the two capsules can be liquid, semi-solid or solid.

For the purposes of the present invention, by extracts are meant, in general, products that are obtained from plants and medicinal herbs of different types. The parts of the plant that can be used to obtain these products are multiple: leaves, flowers, fruits (pulp and/or peel), seeds, roots, rhizomes, bark, etc. They can be preparations in liquid form, solid form (dry extracts) or of intermediate consistency (soft extracts), obtained through suitable extraction processes, preferably through extractions in a hydroalcoholic solvent; or in a supercritical solvent, possibly by resorting to maceration or percolation, or to other suitable procedures.

Preferably, the suitable excipients and/or diluents are those known to the person skilled in the art for the preparation of solid or liquid formulations.

Preferably, the suitable excipients and/or diluents are selected from the group consisting of: gelatin; vegetable polysaccharides (for example hydroxypropyl methylcellulose or HPMC); plasticizers; solvents; binders; disintegrants; lubricants; preservatives; antioxidants; colorants; flavoring agents; buffers; and combinations of the foregoing.

Preferably, examples of excipients and/or diluents are selected from the group consisting of: gelatin; vegetable polysaccharides (for example hydroxypropyl methylcellulose or HPMC); ethyl alcohol; water; silica; magnesium stearate; anhydrous dibasic calcium phosphate; vitamin E acetate; and combinations of the foregoing.

It should be noted that the percentages for the components (active ingredients, excipients/diluents) are calculated considering the content of the capsule, whether it be powder or liquid (oil), therefore not considering the weight of the capsule itself.

Preferably, the capsule-in-capsule composition may optionally contain the band application solution or banding solution (described below) which acts as a further excipient for the composition taken as a whole.

### Outer capsule

Preferably, the outer capsule is a non-gastro-resistant capsule.

Preferably, the outer capsule comprises HPMC, more preferably it further comprises additional ingredients capable of allowing the disintegration of the capsule at acidic pH, for example pectin, or it comprises HPMC chemically modified so as to make it sensitive and disintegrate it at acidic pH.

Preferably, the active ingredients comprised in the outer capsule, i.e. preferably melatonin and essential oil of lavender, are rapidly released in the gastrointestinal tract, producing an immediate effect for relaxation and sleep induction.

Preferably, the mixture comprised in the outer capsule is in liquid form.

Preferably, the mixture comprised in the outer capsule comprises an emulsifier capable of promoting the absorption of the active ingredients.

Preferably, the emulsifier is selected from the group consisting of: polysorbate 80, vegetable lecithins, phospholipids, and mixtures of the foregoing.

Preferably, the emulsifier is comprised in an amount between 1% and 30% by weight, preferably between 2% and 25%, preferably between 5% and 20%, preferably between 10% and 15% by weight, by weight on the weight of the mixture comprised in the outer capsule.

Preferably, the weight ratio of the amounts of melatonin and essential oil of lavender is between 1:10 and 1:300, and wherein the sum of the amounts of melatonin and essential oil is between 10% and 40% by weight on the weight of the mixture comprised in the outer capsule.

More preferably, the weight ratio of the amounts of melatonin and essential oil of lavender is between 1:20 and 1:200, preferably between 1:40 and 1:100, preferably between 1:70 and 1:90 and the sum of the amounts of melatonin and essential oil is between 12% and 35% by weight, preferably between 15% and 35% by weight, preferably between 18% and 32% by weight, preferably between 20% and 30% by weight, preferably between 25% and 30% by weight, on the weight of the mixture comprised in the outer capsule.

Preferably, the outer capsule is of size "00".

Preferably, the mixture comprised in the outer capsule comprises suitable excipients and/or diluents in an amount between 10% and 70% by weight, preferably between 20% and 70% by weight, preferably between 30% and 70% by weight, preferably between 40% and 70% by weight, preferably between 50% and 70% by weight, on the weight of the mixture comprised in the outer capsule.

### Inner capsule

Preferably, the inner gastro-resistant capsule is composed of a material selected from the group consisting of: HPMC, animal gelatin, and mixtures of the foregoing.

Preferably, the inner capsule comprising the black cherry extract comprising procyanidin B2 and the gelling agent is designed to be resistant to gastric acids, protecting the black cherry extract during its passage through the stomach.

Preferably, the inner capsule comprising the black cherry extract may comprise further active components such as vegetable extracts and/or active molecules, such as for example melatonin, essential oil of lavender and/or vitamins, whose bioavailability is increased thanks to the action of procyanidin B2 released at the intestinal level.

Preferably, the mixture comprised in the inner capsule comprises vitamins selected from the group consisting of: vitamin A, vitamins of the B group, vitamin E; and mixtures of the foregoing.

Preferably, the mixture comprised in the inner capsule comprises vitamin B6 (i.e. pyridoxine hydrochloride).

Preferably, vitamin B6 is comprised in an amount between 0.1% and 2% by weight, preferably between 0.25% and 1.5% by weight, preferably between 0.5% and 1% by weight, on the weight of the mixture comprised in the inner capsule.

Preferably, the inner capsule is of size "3".

Preferably, the mixture comprised in the inner capsule comprises suitable excipients and/or diluents in an amount between 5% and 50% by weight, preferably between 5% and 40% by weight, preferably between 5% and 30% by weight, preferably between 5% and 20% by weight, preferably between 5% and 15% by weight, on the weight of the mixture comprised in the inner capsule.

### Melatonin

Melatonin, or N-acetyl-5-methoxytryptamine, is a key hormone in the regulation of the sleep-wake cycle. Endogenous melatonin is produced by the pineal gland; it is also produced by other animal species, by plants and by microorganisms.

Preferably, the melatonin comprised in the composition of the invention is obtained by chemical synthesis processes or, alternatively, by biosynthesis processes comprising microbial fermentation and/or metabolic engineering.

Preferably, melatonin is in an amount between 0.1% and 1% by weight, preferably between 0.15% and 0.75% by weight, preferably between 0.2% and 0.6% by weight, preferably between 0.25% and 0.5% by weight, on the weight of the mixture comprised in the outer capsule.

### Essential oil of lavender

The essential oil of lavender is a liquid extract (or essential oil) available commercially. Preferably, this extract is obtained by distillation or supercritical extraction, which is a known extraction technique whereby the material to be extracted is subjected to certain conditions of temperature and pressure and in the presence of a gas, generally carbon dioxide (CO2).

Preferably, the essential oil of lavender is of food grade, i.e., it conforms to the specifications of food ingredients according to the Codex Alimentarius and European Regulation 925/2023, as well as to European standards on pesticides, residual solvents, and the like.

Preferably, the essential oil of lavender is in an amount between 5% and 50% by weight, preferably between 10% and 40% by weight, preferably between 15% and 35% by weight, preferably between 18% and 32% by weight, preferably between 20% and 30% by weight, on the total weight of the mixture comprised in the outer capsule.

Preferably, the Lavender belongs to a species and/or subspecies and/or variants and/or basionyms selected from the group consisting of: *Lavandula angustifolia Mill., Lavandula angustifolia subsp. pyrenaica (DC.) Guinea, Lavandula heterophylla Viv., Lavandula intermedia Emeric ex Loisel., Lavandula latifolia Medik, Lavandula stoechas L.,* preferably *Lavandula angustifolia Mill.*

The essential oil of lavender is preferably extracted from flowers and flowering tops of the aforementioned species and/or subspecies and/or variants and/or basionyms.

### Black cherry extract comprising procyanidin B2

Preferably, the black cherry extract is obtained from the fruits of the species *Prunus cerasus.*

According to further preferred embodiments, the black cherry extract is obtained from the fruits of the species *Prunus avium, Prunus fruticosa, Prunus serrulata, Prunus serotina.* The Applicant intends to highlight that, for the purposes of the present invention, the phytocomplexes comprised in the extracts of the fruits of *Prunus cerasus* and the above-cited species are comparable in terms of procyanidin B2 content and effects obtained.

For the purposes of the present invention, by phytocomplex is meant the set of substances, pharmacologically active or not, that characterize the plant itself and that represent its chemical composition, from which its pharmacological and toxicological properties derive (Capasso F. "FITOTERAPIA. Impiego razionale delle droghe vegetali" chap.20, page 249, Springer ed.).

Preferably, the process of obtaining the black cherry extract comprises the following steps: separating the fruit pulp from the stone, pressing the pulp to obtain a juice, drying the juice with a conventional procedure known to the person skilled in the art, obtaining a dry black cherry extract.

According to a preferred embodiment, the black cherry extract comprising procyanidin B2 is comprised in an amount between 140 mg and 210 mg, preferably between 160 mg and 210 mg, preferably between 180 mg and 210 mg, or in an amount between 60% and 90% by weight, preferably between 70% and 90% by weight, preferably between 80% and 90% by weight, on the weight of the mixture comprised in the inner capsule.

Proanthocyanidins are polyphenolic compounds, structurally formed by oligomeric repetitions of flavanols such as, for example, catechin, epicatechin and their gallic acid esters; their acid hydrolysis leads to the formation of anthocyanidins.

Procyanidins are molecules belonging to the class of proanthocyanidins composed solely of catechin or epicatechin units. They are classified as A or B depending on which units are connected to each other and how.

Procyanidin B2 is composed of two (-)-epicatechin units linked together by a single C-C bond between the carbon in position 4β of one unit and the carbon in position 8 of the second unit.

Preferably, the black cherry extract comprises procyanidin B2 in an amount between 0.0015% and 0.17% by weight, preferably between 0.002% and 0.15% by weight, preferably between 0.005% and 0.125% by weight, preferably between 0.005% and 0.10% by weight, on the weight of the black cherry extract.

Procyanidin B2 comprised in the black cherry extract is an agent capable of promoting relaxation and capable of improving sleep quality by promoting a deeper and uninterrupted rest.

Moreover, advantageously, the black cherry extract comprising procyanidin B2 comprises plant-derived melatonin intrinsic to the black cherry phytocomplex.

Preferably, the black cherry extract comprises melatonin in an amount between 0.001% and 0.1% by weight, on the weight of the black cherry extract.

### Gelling agent

The gelling agent comprised in the composition is selected from the group consisting of: gellan gum, carrageenan, pectin, sodium alginate, tara gum, cross-linked modified starch, and mixtures of the foregoing. Preferably, the gelling agent comprises or consists of carrageenan.

Preferably, the gelling agent is a natural gelling agent or is a gelling agent of natural origin.

For the purposes of the present invention, by "natural" gelling agent is meant a gelling agent obtained from natural sources (e.g. vegetable, animal, microorganisms) with processes that do not involve chemical reactions.

For the purposes of the present invention, by gelling agent "of natural origin" is meant a gelling agent originally from natural sources obtained with processes that involve chemical reactions.

It should be noted that, for the purposes of the present invention, the gelling agent is not to be considered as a mere excipient and/or diluent, as it exerts a fundamental action in controlling the release of the active ingredients comprised in the inner capsule.

Preferably, the gelling agent is in an amount between 1% and 15% by weight, preferably between 2% and 10% by weight, preferably between 2.5% and 8% by weight, preferably between 3% and 7% by weight, on the weight of the mixture comprised in the inner capsule.

According to a preferred embodiment, the weight ratio between gelling agent, preferably carrageenan, and black cherry extract comprising procyanidin B2 is between 1:4 and 1:90, preferably between 1:6 and 1:80, preferably between 1:8 and 1:70, preferably between 1:10 and 1:60, preferably between 1:10 and 1:50, preferably between 1:10 and 1:40, preferably between 1:10 and1:30.

The Applicant wishes to emphasize that the combination of the black cherry extract comprising procyanidin B2 with a gelling agent, preferably carrageenan, allows for the gradual and controlled release of procyanidin B2 in the enteric tract. In fact, the gelling agent is a hydrophilic polymer which, once released into the intestine following the disintegration of the gastro-resistant inner capsule, forms a matrix comprising the active ingredient, i.e. the black cherry extract comprising procyanidin B2, which by slowly dissolving releases the active ingredient in a controlled manner.

### Band application solution or "banding" solution

Preferably, in the pharmaceutical context, by band application solution (in English "banding solution") is meant a solution used to apply a band (often composed of a binder or a protective film) on capsules to improve their stability, protection (coating or sealing for the protection of the capsule content or for release control) or aesthetic appearance. It is known that the band application solution for capsules is composed of a mixture of ingredients (excipients/diluents) that act as binders, solubilizers and, sometimes, also of additives that improve the adhesion or protection of the coating.

For the purposes of the invention, suitable excipients and/or diluents for the band application solution are selected from the group consisting of: modified cellulose derivative, for example HPMC; solvents, such as water and/or ethyl alcohol. Preferably, the band application solution is in an amount ≤ 1%by weight, preferably between 0.2% and 0.9% by weight, preferably between 0.5% and 0.7% by weight, on the total weight of the solid formulation taken as a whole.

### Forms of the composition object of the invention

Preferably, the composition according to the present invention is in the form of a nutraceutical composition or a food supplement.

By nutraceutical composition is meant a composition comprising a food derivative ("nutraceutical") to which, in addition to the basic nutritional value, the ability to optimize physiological functions is attributed.

By food supplement is meant a formulation that falls within the definition underlying Directive 2002/46/EC and subsequent amendments. In this legislation, food supplements are defined as: "foodstuffs intended to supplement the common diet and which constitute a concentrated source of nutrients, such as vitamins and minerals, or of other substances having a nutritional or physiological effect, in particular, but not exclusively, amino acids, essential fatty acids, fibers and extracts of vegetable origin, both single-compound and multi-compound, in pre-dosed forms".

### Uses

An object of the present invention is a composition according to the invention, preferably as a nutraceutical composition or as a food supplement, for use in the treatment or prevention of mild insomnia, sleep disorders and anxiety disorders.

For the purposes of the present invention, by mild insomnia is meant an insomnia disorder of mild entity or intensity. The symptomatology related to insomnia can be nocturnal, such as difficulty falling asleep, frequent or prolonged nocturnal awakenings, early morning awakenings, or diurnal, such as a feeling of fatigue and low energy, difficulty with attention, concentration and memory, mood disorders, difficulty at work or in study.

By sleep disorders are meant disorders that negatively affect the sleep-wake cycle, such as parasomnias and dyssomnias like, for example, circadian rhythm disorders, narcolepsy, nightmare disorder, REM sleep behavior disorder.

By anxiety disorders are meant states of worry of variable duration or frequency. They are caused by a feeling of agitation about events or daily problems and are characterized by the thought of being inadequate to face them. Anxiety disorders manifest especially in situations of difficulty related to daily life (work, family, daily commitments or financial problems). Such disorders generate a series of symptoms, such as restlessness, constant worries, disturbed sleep and bad mood, which can have an impact on the quality of life.

A further object of the present invention is the non-therapeutic use of the composition according to the invention, preferably in the form of a nutraceutical composition or a food supplement, for promoting relaxation, decreasing stress, improving sleep quality, and promoting psychophysical well-being.

For the purposes of the present invention, by promoting relaxation is meant supporting the modulation of stress and the regulation of emotional tone through a mild action operated by active ingredients of natural origin on the nervous system.

By decreasing stress is meant promoting a reduction of symptoms related to stress such as, for example, anxiety, muscle tension, irritability, difficulty in concentration.

By improving sleep quality is meant facilitating and speeding up the sleep induction phase and allowing for continuous sleep, in the absence of nocturnal awakenings.

For the purposes of the present invention, by promoting psychophysical well-being is meant allowing, mainly through the regulation of the circadian rhythm, a regulation of mood, of the stress response and of the general tone, promoting a general state of balance and health.

Preferably, the active ingredients present in the outer capsule are released at the gastric level to promote sleep induction and the inner capsule disintegrates at intestinal pH, releasing the black cherry extract comprising procyanidin B2 at controlled times.

### Preparation process

Preferably, the process for obtaining the composition according to the invention in capsule-in-capsule form comprises the following phases:
a) obtaining the inner capsule, phase a) comprising the following sub-phases:
   a1) weighing and mixing the active ingredients and the suitable excipients and/or diluents comprised in the mixture comprised in the inner capsule, obtaining said mixture, preferably in powder form;
   a2) encapsulating the mixture by means of an encapsulator known to the person skilled in the art, obtaining the inner capsule;
b) filling the outer capsule, phase b) comprising the following sub-phases:
   b1) arranging the inner capsule inside the open outer capsule;
   b2) weighing and mixing the active ingredients and the suitable excipients and/or diluents comprised in the mixture comprised in the outer capsule, obtaining said mixture, preferably in liquid form;
   b3) arranging the mixture inside the open outer capsule by means of a nozzle;
c) sealing the outer capsule, phase c) comprising the following sub-phases:
   c1) closing the outer capsule;
   c2) sealing the outer capsule by means of a band application solution (banding solution).

### EXAMPLES

Hereinafter, illustrative and non-limiting examples of the invention are reported.

### Example 1 - Composition in capsule-in-capsule form

| **Inner gastro-resistant capsule "3"** | | |
|---|---|---|
| *Components* | *mg*/*cps* | *mg active*/*cps* |
| Black cherry extract | 200,000 | 0,34 mg poliphenols (comprising 0,03 mg anthocyanidins) |
| Pyridoxine hydrochloride (vitamin B6, tit. 82.27%) | 1,957 | 1,40 |
| Aerosil 200 f (micronized silica) | 2,200 | |
| Magnesium stearate | 3,500 | |
| Carrageenan (E407) | 10,000 | |
| Anhydrous dibasic calcium phosphate | 12,343 | |
| ***Powder weight*** | ***230,000*** | |
| HPMC Capsule #3 Gastro-resistant | 50,000 | |
| ***Total weight inner gastro-resistant capsule*** | ***280,000*** | |

| **Outer capsule (non-gastro-resistant) "00"** | |
|---|---|
| *Components* | *mg*/*cps* |
| Sunflower oil | 174,9899 |
| Oily vitamin e acetate | 4,0000 |
| Essential oil of lavender | 80,0000 |
| Melatonin (tit. 99%) | 1,0101 * |
| Polysorbate 80 | 40,0000 |
| ***Liquid weight*** | ***300,0000*** |
| Capsule in transparent HPMC #00 | 130,0000 |
| ***Total weight outer capsule*** | ***430,0000*** |

| **Banding solution (transparent "1")** | |
|---|---|
| *Components* | *g*/*cps* |
| Production water | 1,344 |
| Banding kit for transparent HPMC capsules | 0,640 |
| Ethyl alcohol 96.4-96.5° | 2,016 |
| ***Total weight banding solution*** | ***4,000*** |

| | |
|---|---|
| *Note that 1.0101 mg of melatonin (tit. 99%) correspond to 1.0000 mg of pure melatonin (tit. 100%). | |

### BIBLIOGRAPHY

[1] Chung, J., Choi, M., & Lee, K. (2022). Effects of Short-Term Intake of Montmorency Tart Cherry Juice on Sleep Quality after Intermittent Exercise in Elite Female Field Hockey Players: A Randomized Controlled Trial. International Journal of Environmental Research and Public Health, 19(16), 10272. DOI: 10.3390/ijerph191610272
[2] Losso, J. N., Finley, J. W., Karki, N., Liu, A. G., Prudente, A., Tipton, R., & Greenway, F. L. (2017). Pilot Study of the Tart Cherry Juice for the Treatment of Insomnia and Investigation of Mechanisms. American Journal of Therapeutics, 24(3), e344-e350. DOI: 10.1097/MJT.0000000000000584
[3] Howatson, G., Bell, P. G., Tallent, J., Middleton, B., McHugh, M. P., & Ellis, J. (2012). Effect of tart cherry juice (Prunus cerasus) on melatonin levels and enhanced sleep quality. European Journal of Nutrition, 51(8), 909-916. DOI: 10.1007/s00394-011-0263-7
[4] Butterweck, V., Liefländer-Wulf, U., Winterhoff, H., & Nahrstedt, A. (2003). Plasma levels of hypericin in presence of procyanidin B2 and hyperoside: a pharmacokinetic study in rats. Planta Med., 69, 189-192.
[5] Yang, K., & Chan, C. B. (2017). Proposed mechanisms of the effects of proanthocyanidins on glucose homeostasis. Nutrition Reviews, 75(8), 642-657. DOI: 10.1093/nutrit/nux028

## Claims

1. Composition comprising:
• melatonin;
• essential oil of lavender;
• black cherry extract comprising procyanidin B2;
wherein:
- the composition is in capsule-in-capsule form, wherein an inner gastro-resistant capsule is arranged inside a non-gastro-resistant outer capsule;
- the non-gastro-resistant outer capsule comprises a mixture comprising lavender essential oil, melatonin and suitable excipients and/or diluents;
- the inner gastro-resistant capsule comprises a mixture comprising the black cherry extract, a gelling agent selected from the group consisting of: gellan gum, carrageenan, pectin, and mixtures of the foregoing, preferably the gelling agent comprises or consists of carrageenan, and suitable excipients and/or diluents.

2. Composition according to claim 1, wherein the weight ratio of the amounts of melatonin and lavender essential oil is between 1:10 and 1:300, and wherein the sum of the amounts of melatonin and lavender essential oil is between 10% and 40% by weight on the weight of the mixture comprised in the outer capsule.

3. Composition according to claims 1 or 2, wherein the black cherry extract is in an amount between 140 mg and 210 mg or in an amount between 60% and 90% by weight, based on the weight of the mixture in the inner capsule.

4. Composition according to any one of claims 1 to 3, wherein the black cherry extract comprises procyanidin B2 in an amount between 0.0015% and 0.17% by weight, based on the weight of the black cherry extract.

5. Composition according to any one of claims 1 to 4, wherein the weight ratio between gelling agent, preferably carrageenan, and black cherry extract comprising procyanidin B2 is between 1:4 and 1:90.

6. Composition according to any one of claims 1 to 5, in the form of a food supplement or in the form of a nutraceutical composition.

7. Composition according to claim 6 for use in the treatment or prevention of mild insomnia, sleep disorders and anxiety disorders.

8. Non-therapeutic use of the composition according to claim 6 for promoting relaxation, decreasing stress, improving sleep quality, and promoting psychophysical well-being.

9. Composition for use according to claim 7 or non-therapeutic use of the composition according to claim 8, wherein the active ingredients present in the outer capsule are released at the gastric level to promote sleep induction and the inner capsule disintegrates at intestinal pH, releasing the black cherry extract comprising procyanidin B2 at controlled times.
